# EUROPEAN PATENT APPLICATION

(11) **EP 4 831 617 A1**
(43) Date of publication of application: **28.10.2026**
(21) Application number: 25834103.1
(22) Date of filing: 11.03.2025
(51) Int. Cl.: G01N 21/31

(54) **METHOD FOR CALIBRATING POTENCY OF HAINA POLYSACCHARIDE**

(30) Priority: 06.03.2025 CN 202510261711
(71) Applicant: Harbin Hongdoushan Bio-pharm Co., Ltd., Harbin, Heilongjiang 150600 (CN)
(72) Inventor: HUANG, Xueshi, Harbin, Heilongjiang 150600 (CN); HAN, Li, Harbin, Heilongjiang 150600 (CN); WANG, Yicheng, Harbin, Heilongjiang 150600 (CN); WANG, Zhiguo, Harbin, Heilongjiang 150600 (CN); WU, Xuechai, Harbin, Heilongjiang 150600 (CN); MENG, Yiqiang, Harbin, Heilongjiang 150600 (CN)
(74) Representative: LEITZINGER OY
(86) International application number: PCT/CN2025/081746
(87) International publication number: WO 2026/183869

(57) **Abstract**

A method for potency determination of a haina polysaccharide (HPS) is provided, relating to the technical field of pharmaceuticals. The method includes the following steps: obtaining an HPS activity curve by a p-nitroaniline chromogenic assay, where the HPS activity curve uses a reciprocal of an HPS weight as an abscissa and a logarithm of a residual Factor VIII (FVIII) activity unit as an ordinate; and defining a weight of the HPS that inhibits an Intrinsic Factor Xase (iFXase) activity required for formation of 1 IU of an FVIII as one potency unit, and obtaining a potency of the HPS from the HPS activity curve. The method for potency determination of the HPS enables accurate determination of an anticoagulant activity index of the HPS, and is of great significance in ensuring drug efficacy, medication safety, and quality control.

## Description

### TECHNICAL FIELD

The present disclosure relates to the technical field of pharmaceuticals, and in particular to a method for potency determination of a haina polysaccharide (HPS).

### BACKGROUND

Sea cucumbers, in their body wall, is rich in polysaccharides, primarily including two major categories: fucoidan and fucosylated chondroitin sulfate. Among these, sea cucumber-derived fucosylated chondroitin sulfate demonstrates a wide range of biological activities due to its unique structure, such as lipid-lowering, antitumor, antiviral, and anti-inflammatory effects. Notably, this component possesses excellent anticoagulant and antithrombotic activities.

Potency of a drug refers to a dose or a concentration required for the drug to achieve a specific effect, serving as a critical indicator for measuring the intensity of a drug's action and reflecting the affinity and intrinsic activity of the drug with a receptor. Even for drugs produced by the same manufacturer, variations in production processes and raw material sources may lead to differences in actual activity between different batches. Through potency determination, an activity index of the drug could be accurately determined, ensuring that clinicians could prescribe appropriate dosages based on precise potency, thereby enabling effective treatment for patients.

HPS is a type of fucosylated chondroitin sulfate-based natural polysaccharide extracted from the body wall of the sea cucumber. The HPS targets an endogenous coagulation pathway and exhibits excellent anticoagulant activity. Currently, there are no marketed drugs with the same target of action. Therefore, it is necessary to establish a method for potency determination of an HPS reference standard to accurately determine the activity index of the HPS.

### SUMMARY

In view of this, the present disclosure provides a method for potency determination of a HPS. The method for potency determination of the HPS enables accurate determination of an anticoagulant activity index of the HPS, and is of great significance in ensuring drug efficacy, medication safety, and quality control.

To achieve the above object, the present disclosure provides the following technical solutions.

The present disclosure provides a method for potency determination of an HPS, including the following steps:
obtaining an HPS activity curve by a p-nitroaniline chromogenic assay, where the HPS activity curve uses a reciprocal of an HPS weight as an abscissa and a logarithm of a residual Factor VIII (FVIII) activity unit as an ordinate; and
defining a weight of the HPS that inhibits an Intrinsic Factor Xase (iFXase) activity required for formation of 1 IU of an FVIII as one potency unit, and obtaining a potency of the HPS from the HPS activity curve; where
the HPS is a fucosylated chondroitin sulfate polysaccharide; the HPS has a weight-average molecular weight of 90,000 to 130,000, and has a monosaccharide composition including glucuronic acid, N-acetyl-D-galactosamine, and fucose, where a molar ratio of the glucuronic acid, the *N*-acetyl-*D*-galactosamine, and the fucose is in a range of 1 : (0.8-1.2) : (0.5-0.8); and a sulfate group in the HPS has a mass percentage of 25% to 40%.

In some embodiments, the obtaining the HPS activity curve includes:
preparing HPS working solutions with gradient concentrations;
mixing the HPS working solutions, an FVIII solution, and an activation reagent solution, and conducting a first incubation to obtain a first incubation mixture, where the activation reagent solution includes a Factor IXa (FIXa), a Factor IIa (Thrombin) (FIIa), a phospholipid, a buffer, and CaCl₂;
mixing the first incubation mixture with a Factor X (FX) solution, and conducting a second incubation to obtain a second incubation mixture;
mixing the second incubation mixture with a chromogenic substrate solution, conducting a third incubation, adding a stop solution, and measuring an absorbance of a resulting mixture at 405 nm to obtain an absorbance value, where a chromogenic substrate in the chromogenic substrate solution is SXa-11 (Sequence: Suc-Ile-Glu-(Pip)Gly-Arg-pNA, HCl); and
substituting the absorbance value into an FVIII complex activity standard curve, calculating the residual FVIII activity unit, and plotting the HPS activity curve using the reciprocal of the HPS weight as the abscissa and the logarithm of the residual FVIII activity unit as the ordinate; where the FVIII complex activity standard curve uses a logarithm of an FVIII concentration as an abscissa and the absorbance value as an ordinate.

In some embodiments, the first incubation is conducted at 37°C for 5 minutes to 20 minutes.

In some embodiments, the second incubation is conducted at 37°C for 1 minute to 10 minutes.

In some embodiments, the third incubation is conducted at 37°C for 10 minutes to 30 minutes.

In some embodiments, the stop solution is a citric acid solution having a concentration of 0.01 g/mL to 0.1 g/mL.

In some embodiments, the HPS working solutions each have a concentration of 328 ng/mL to 500 ng/mL.

In some embodiments, the buffer in the activation reagent solution is Tris(hydroxymethyl)aminomethane hydrochloride (Tris-HCl).

In some embodiments, the FVIII complex activity standard curve is obtained by a process including:
preparing FVIII standard solutions with gradient concentrations;
mixing the FVIII standard solutions, water, and the activation reagent solution, and conducting a fourth incubation to obtain a fourth incubation mixture, where the activation reagent solution includes the FIXa, the FIIa, the phospholipid, the buffer, and the CaCl₂;
mixing the fourth incubation mixture with the FX solution, and conducting a fifth incubation to obtain a fifth incubation mixture;
mixing the fifth incubation mixture with the chromogenic substrate solution, conducting a sixth incubation, adding the stop solution, and measuring an absorbance of an obtained mixture at 405 nm to obtain the absorbance value, where the chromogenic substrate is the SXa-11; and
plotting the FVIII complex activity standard curve using the logarithm of the FVIII concentration as the abscissa and the absorbance value as the ordinate.

In some embodiments, the FVIII standard solutions each have a concentration of 0.95 IU/mL to 3 IU/mL.

The present disclosure provides a method for potency determination of an HPS, including the following steps: obtaining an HPS activity curve by a p-nitroaniline chromogenic assay, where the HPS activity curve uses a reciprocal of an HPS weight as an abscissa, and a logarithm of a residual FVIII activity unit as an ordinate; and defining a weight of the HPS that inhibits an Intrinsic Factor Xase (iFXase) activity required for formation of 1 IU of an FVIII as one potency unit, and obtaining a potency of the HPS from the HPS activity curve. The pharmacological action of the HPS primarily manifests as that of a non-heparin-like anticoagulant drug. A mechanism of action involves selective inhibition of a terminal rate-limiting enzyme of an endogenous coagulation pathway, the "Intrinsic Factor Xase (Intrinsic Tenase, FIXa-FVIIIa-PL-Ca²⁺ complex, iFXase)". In the present disclosure, the potency determination is conducted by measuring the activity of the HPS in inhibiting the iFXase. In a presence of phospholipid (PLP) and calcium ions, the FVIII is activated by thrombin to form an FVIIIa. The FVIIIa, an FIXa, the phospholipid, and the calcium ions then form the iFXase. The iFXase could activate a Factor X (FX) to form an FXa. The FXa could hydrolyzes a chromogenic substrate SXa-11, releasing p-nitroaniline (pNA). The pNA exhibits light absorption at 405 nm, and an amount of the pNA is directly proportional to an absorbance at 405 nm. Based on the above principle, the p-nitroaniline chromogenic assay is adopted to obtain the HPS activity curve. The weight of the HPS that inhibits the iFXase activity required for the formation of 1 IU of the FVIII is defined as one potency unit. The potency of the HPS is then calculated from the HPS activity curve. The method for the potency determination of the HPS enables accurate determination of an anticoagulant activity index of the HPS, and is of great significance in ensuring drug efficacy, medication safety, and quality control.

### BRIEF DESCRIPTION OF THE DRAWINGS

FIG. 1 shows a schematic diagram of the detection mechanism of an FVIII activity assay kit;
FIG. 2 shows an FVIII complex activity standard curve obtained from a first experiment by Operator A;
FIG. 3 shows an HPS activity curve obtained from the first experiment by the Operator A;
FIG. 4 shows an FVIII complex activity standard curve obtained from a second experiment by the Operator A;
FIG. 5 shows an HPS activity curve obtained from the second experiment by the Operator A;
FIG. 6 shows an FVIII complex activity standard curve obtained from a first experiment by Operator B;
FIG. 7 shows an HPS activity curve obtained from the first experiment by the Operator B;
FIG. 8 shows an FVIII complex activity standard curve obtained from a second experiment by the Operator B; and
FIG. 9 shows an HPS activity curve obtained from the second experiment by the Operator B.

### DETAILED DESCRIPTION OF THE EMBODIMENTS

First, a description of the HPS is provided.

In the present disclosure, the HPS refers to a natural polysaccharide extracted from a body wall of sea cucumbers. The sea cucumbers include, but are not limited to, at least one of *Holothuria leucospilota* (Brandt), *Holothuria scabra*, *Stichopus japonicus, Holothuria atra*, and *Holothuria nobilis,* preferably the *Holothuria leucospilota* (Brandt). The HPS is a fucosylated chondroitin sulfate polysaccharide. The HPS has a weight-average molecular weight of 90,000 to 130,000, has a polydispersity index (PDI) of less than or equal to 2, and has a monosaccharide composition including glucuronic acid, N-acetyl-D-galactosamine, and fucose, where a molar ratio of the glucuronic acid, the N-acetyl-D-galactosamine, and the fucose is in a range of 1 : (0.8-1.2) : (0.5-0.8); and a sulfate group in the HPS has a mass percentage of 25% to 40%.

A structural unit of the HPS is shown in Formula I: 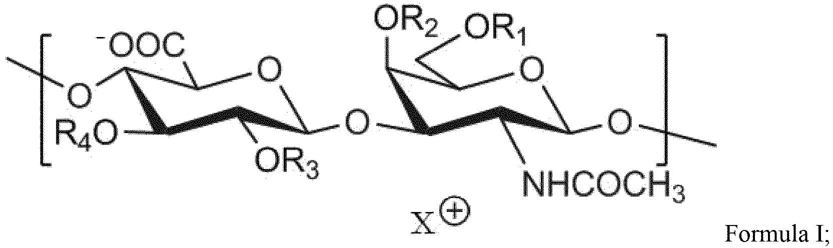
where, in the Formula I, R₁ is selected from the group consisting of H, SO₃⁻, and fucosyl; R₂ is selected from the group consisting of H and SO₃⁻; R₃ is selected from the group consisting of H, and SO₃⁻; R₄ is selected from the group consisting of H, and fucosyl; at least one of R₁ and R₄ is the fucosyl; and X^{⊕} represents a cation;
a structure of the fucosyl in R₁ and R₄ is shown in Formula I-1: 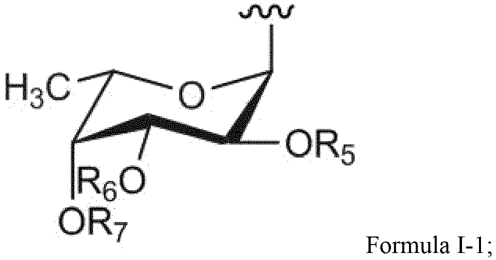
where, in the Formula I-1, R₅ is selected from the group consisting of H, and SO₃⁻; R₆ is selected from the group consisting of H, and SO₃⁻; and R₇ is selected from the group consisting of H, and SO₃⁻.

In the Formula I, the cation is selected from the group consisting of a hydrogen ion, an alkali metal ion, an alkaline earth metal ion, an ammonium ion, and an iron ion, specifically the hydrogen ion, potassium ion, sodium ion, calcium ion, the ammonium ion, and the iron ion, and preferably the sodium ion. When the cation is the sodium ion, the sodium ion in the HPS has a mass percentage of 5% to 15%.

The HPS is a natural fucosylated chondroitin sulfate polysaccharide obtained by the inventors from a body wall of sea cucumbers, which could target a terminal rate-limiting enzyme iFXase in an endogenous coagulation pathway. The HPS provides excellent anticoagulant effects with minimal bleeding side effects, thereby achieving effective anticoagulation without causing bleeding.

Evaluation results of anticoagulant activity of the HPS in mice *in vivo* indicate that the HPS could significantly prolong an activated partial thromboplastin time (APTT) in the mice and rats, without affecting a prothrombin time (PT) and a thrombin time (TT). These results suggest that the HPS targets the endogenous coagulation pathway and does not affect an exogenous coagulation pathway.

Referring to the Chinese Pharmacopoeia General Chapter 1208 "heparin biological assay method", tests are conducted on anti-Factor IIa activity, anti-Factor Xa activity, AT-III-IIa activity, and AT-III-Xa activity of the HPS. Results show that at an experimental dose of 500 µg/mL, the HPS displays no activity against a Factor IIa, a Factor Xa, or an AT-III-IIa, and only shows weak activity against the AT-III-Xa (inhibition rate 36.7%). An activity testing is conducted using enzyme-linked immunosorbent assay (ELISA) on the rate-limiting enzyme "iFXase" of the endogenous coagulation pathway. Results indicate that the HPS could significantly inhibit the rate-limiting enzyme iFXase of the endogenous coagulation pathway, with an IC₅₀ value of 207.5 ng/mL. The above experiments further clarify, mechanistically, that the HPS could selectively inhibit the activity of the rate-limiting enzyme iFXase of the endogenous coagulation pathway, targeting the endogenous coagulation pathway.

In the present disclosure, a method for potency determination specifically refers to a method for determining a potency of an HPS reference standard. The method for potency determination is described in detail below.

The present disclosure provides a method for potency determination of an HPS, including the following steps:
obtaining an HPS activity curve by a *p*-nitroaniline chromogenic assay, where the HPS activity curve uses a reciprocal of an HPS weight an abscissa and a logarithm of a residual FVIII activity unit as an ordinate; and
defining a weight of the HPS that inhibits an Intrinsic Factor Xase (iFXase) activity required for formation of 1 IU of an FVIII as one potency unit, and obtaining a potency of the HPS from the HPS activity curve.

In the present disclosure, the obtaining the HPS activity curve includes:
preparing HPS working solutions with gradient concentrations;
mixing the HPS working solutions, an FVIII solution, and an activation reagent solution, and conducting a first incubation to obtain a first incubation mixture, where the activation reagent solution includes an FIXa, an FIIa, a phospholipid, a buffer, and CaCl₂;
mixing the first incubation mixture with an FX solution, and conducting a second incubation to obtain a second incubation mixture;
mixing the second incubation mixture with a chromogenic substrate solution, conducting a third incubation, adding a stop solution, and measuring an absorbance of a resulting mixture at 405 nm to obtain an absorbance value, where a chromogenic substrate is SXa-11; and
substituting the absorbance value into an FVIII complex activity standard curve, calculating the residual FVIII activity unit, and plotting a standard curve using the reciprocal of the HPS weight as the abscissa and the logarithm of the residual FVIII activity unit as the ordinate; where the FVIII complex activity standard curve uses a logarithm of an FVIII concentration as an abscissa and the absorbance value as an ordinate.

In the present disclosure, a kit used for obtaining the HPS activity curve by the p-nitroaniline chromogenic assay is an FVIII activity assay kit (specification: BIOPHEN FVIII:C). Reagents in the FVIII activity assay kit include R1, R2, R3, and R4, where the R1 is FX, the R2 is an activation reagent including an FIXa, an FIIa, a phospholipid, a buffer, and CaCl₂, and the buffer is Tris-HCl; the R3 is a chromogenic substrate SXa-11; and the R4 is Tris-BSA Buffer, used for diluting an FVIII standard solution.

In some embodiments of the present disclosure, preparation processes of various solutions used in the *p*-nitroaniline chromogenic assay include:
adding ultrapure water to the R1, the R2, and the R3 separately to obtain the FX solution, the activation reagent solution, and the chromogenic substrate solution; where the FX solution has a concentration of 40 nM to 60 nM, and preferably 50 nM; the activation reagent solution includes the FIXa at a concentration of 40 nM to 80 nM, and preferably 60 nM; the FIIa at a concentration of 30 nM to 60 nM, and preferably 47 nM; the phospholipid at a concentration of 0.1 mg/mL to 0.6 mg/mL, and preferably 0.32 mg/mL; and the CaCl₂ is preferably 5-15 mM, and preferably 12 mM. The chromogenic substrate solution has a concentration of 4 mM to 12 mM, and preferably 8.4 mM; and the FVIII solution is obtained by dissolving and diluting an FVIII using the R4.

In the present disclosure, HPS working solutions with gradient concentrations are prepared. In some embodiments of the present disclosure, the HPS working solutions each have a concentration of 328 ng/mL to 500 ng/mL, preferably 328.05 ng/mL to 500 ng/mL, and specifically 328.05 ng/mL, 364.5 ng/mL, 405 ng/mL, 450 ng/mL, or 500 ng/mL; a solvent for preparing the HPS working solutions is water, and the water is double-distilled water (ddH₂O).

In the present disclosure, after obtaining the HPS working solutions, the HPS working solutions, an FVIII solution, and an activation reagent solution are mixed, and a resulting mixture is subjected to a first incubation to obtain a first incubation mixture, where the activation reagent solution includes an FIXa, an FIIa, a phospholipid, a buffer, and CaCl₂. In some embodiments of the present disclosure, the FVIII solution has a concentration of 3 IU/mL; a volume ratio of the HPS working solutions to the FVIII solution is 1 : 1; a volume ratio of the HPS working solutions to the activation reagent solution is 1 : 1; the first incubation is conducted at 37°C for 5 minutes to 20 minutes, and specifically 15 minutes. During the first incubation, in a presence of the phospholipid (PLP) and calcium ions, an FVIII is activated by the FIIa to form an FVIIIa, and the FVIIIa, the FIXa, the phospholipid, and the calcium ions form the iFXase.

In the present disclosure, after the first incubation mixture is obtained, the first incubation mixture is mixed with an FX solution, and a resulting mixture is subjected to a second incubation to obtain a second incubation mixture. In some embodiments of the present disclosure, a volume ratio of the HPS working solution to the FX solution is 1 : 1; the second incubation is conducted at 37°C for 1 minute to 10 minutes, and preferably 1 minute. During the second incubation, the iFXase activates the FX to form FXa, while simultaneously, due to a presence of the HPS, activity of a part of the iFXase is inhibited, preventing the iFXase from activating the FX.

In the present disclosure, after the second incubation mixture is obtained, the second incubation mixture is mixed with a chromogenic substrate solution, an obtained mixture is subjected to a third incubation, a stop solution is added, and an absorbance of a resulting mixture at 405 nm is measured to obtain an absorbance value. In some embodiments of the present disclosure, a chromogenic substrate in the chromogenic substrate solution is SXa-11; a volume ratio of the HPS working solutions to the chromogenic substrate solution is 1 : 1; the third incubation is conducted at 37°C for 10 minutes to 30 minutes, and specifically 15 minutes; the stop solution is a citric acid solution, and the citric acid solution has a concentration of 0.01 g/mL to 0.1 g/mL, and specifically 0.02 g/mL; a volume ratio of the HPS working solutions to the citric acid solution is 2 : 3. In some embodiments of the present disclosure, an instrument used for measuring the absorbance is a microplate reader. During the third incubation, the FXa hydrolyzes the chromogenic substrate SXa-11, releasing p-nitroaniline (pNA); the pNA exhibits light absorption at 405 nm, and an amount of the pNA is proportional to the absorbance value at 405 nm.

In the present disclosure, the absorbance value is substituted into an FVIII complex activity standard curve, the residual FVIII activity unit is calculated, and the HPS activity curve is plotted using the reciprocal of the HPS weight as the abscissa and the logarithm of the residual FVIII activity unit as the ordinate. In the present disclosure, after the HPS activity curve is obtained, the method further includes obtaining a fitting equation based on the HPS activity curve.

In the present disclosure, the FVIII complex activity standard curve uses a logarithm of an FVIII concentration as an abscissa and the absorbance as an ordinate.

In some embodiments of the present disclosure, the FVIII complex activity standard curve is obtained by a process including:
preparing FVIII standard solutions with gradient concentrations;
mixing the FVIII standard solutions, water, and the activation reagent solution, and conducting a fourth incubation to obtain a fourth incubation mixture, where the activation reagent solution includes the FIXa, the FIIa, the phospholipid, the buffer, and the CaCl₂;
mixing the fourth incubation mixture with the FX solution, and conducting a fifth incubation to obtain a fifth incubation mixture;
mixing the fifth incubation mixture with the chromogenic substrate solution, conducting a sixth incubation, adding the stop solution, and measuring an absorbance of an obtained mixture at 405 nm to obtain the absorbance value, where the chromogenic substrate is the SXa-11; and
plotting the FVIII complex activity standard curve using the logarithm of the FVIII concentration as the abscissa and the absorbance value as the ordinate.

In the present disclosure, a kit used for obtaining the FVIII complex activity standard curve is the same as that used for obtaining the HPS activity curve. Preparation methods for the FX solution, the activation reagent solution, and the chromogenic substrate solution are also the same as those used in an HPS activity curve testing and will not be reiterated here.

In the present disclosure, FVIII standard solutions with gradient concentrations are prepared. In some embodiments of the present disclosure, the FVIII standard solutions each have a concentration of 0.95 IU/mL to 3 IU/mL, and preferably 0.94922 IU/mL to 3 IU/mL. Specific concentrations of the FVIII standard solutions with gradient concentrations are 3 IU/mL, 2.25 IU/mL, 1.6875 IU/mL, 1.265625 IU/mL, and 0.94922 IU/mL. In specific embodiments of the present disclosure, a reagent R4 from the kit is used to dissolve the FVIII to prepare a 4 IU/mL stock solution, and the stock solution is then serially diluted using the reagent R4 to obtain the FVIII standard solutions with gradient concentrations.

In the present disclosure, after the FVIII standard solutions are obtained, the FVIII standard solutions, water, and the activation reagent solution are mixed, and a resulting mixture is subjected to a fourth incubation to obtain a fourth incubation mixture. In some embodiments of the present disclosure, the fourth incubation is conducted at 37°C for 5 minutes to 20 minutes, and specifically 15 minutes. Specific operating conditions for the fourth incubation are consistent with those of the first incubation, except that the HPS working solution is replaced with water and the FVIII solution is replaced with the FVIII standard solutions with gradient concentrations.

In the present disclosure, after the fourth incubation mixture is obtained, the fourth incubation mixture is mixed with the FX solution, and a resulting mixture is subjected to a fifth incubation to obtain a fifth incubation mixture. In some embodiments of the present disclosure, the fifth incubation is conducted at 37°C for 1 minute to 10 minutes, and specifically 1 minute. Specific operating conditions for the fifth incubation are consistent with those of the second incubation and will not be reiterated here.

In the present disclosure, after the fifth incubation mixture is obtained, the fifth incubation mixture is mixed with the chromogenic substrate solution, a resulting mixture is subjected to a sixth incubation, the stop solution is added, and an absorbance of an obtained mixture at 405 nm is measured to obtain an absorbance value, where the chromogenic substrate is SXa-11. In some embodiments of the present disclosure, the sixth incubation is conducted at 37°C for 10 minutes to 30 minutes, and specifically 15 minutes. Specific operating conditions for the sixth incubation are consistent with those of the third incubation and will not be reiterated here.

In the present disclosure, after the absorbance value is obtained, the FVIII complex activity standard curve is plotted using the logarithm of the FVIII concentration as the abscissa and the absorbance value as the ordinate. In specific embodiments of the present disclosure, after the FVIII complex activity standard curve is obtained, the method further includes obtaining a fitting equation based on the FVIII complex activity standard curve.

In the present disclosure, the absorbance value obtained from the HPS activity curve is substituted into the FVIII complex activity standard curve to calculate a corresponding FVIII concentration for that absorbance value. This FVIII concentration is a residual FVIII concentration in the HPS activity curve. The residual FVIII activity unit is further calculated based on the residual FVIII concentration. The HPS activity curve is then plotted using the logarithm of the residual FVIII activity unit and the corresponding absorbance value. Subsequently, based on the HPS activity curve, the weight of HPS that inhibits the iFXase activity required for formation of 1 IU of the FVIII is calculated, which is defined as one potency unit of the HPS. For convenience of expression, the potency of the HPS is ultimately represented as a number of potency units per unit mass of polysaccharide, where the unit mass is 1 mg. In specific embodiments of the present disclosure, the potency of the HPS is tested 4 times, and an average of the 4 tests is taken as the potency of the HPS.

The following clearly and completely describes the technical solutions of the present disclosure with reference to the examples of the present disclosure. Apparently, the described examples are merely a part rather than all of the embodiments of the present disclosure. All other embodiments obtained by a person of ordinary skill in the art based on the examples of the present disclosure without creative efforts shall fall within the scope of the present disclosure.

### Example 1 Extraction of HPS

Body walls of *Holothuria leucospilota* (Brandt) were carefully separated from other tissues and immediately immersed in acetone, then stored at 4°C for 24 hours. A dried tissue (500 g) was ground into a powder and suspended in 10 L of 0.1 mol/L sodium acetate buffer (pH=6) containing 50 g of papain. A resulting suspension was subjected to incubation at 65°C for 24 hours. A resulting incubation mixture was then centrifuged at a low temperature (4,000 rpm, 4°C for 30 minutes) to obtain an enzymatic hydrolysate supernatant. A 6 mol/L aqueous hydrochloric acid solution was added to the enzymatic hydrolysate supernatant to adjust a pH value to 2.5 ± 0.5. A resulting mixture was subjected to a reaction for 2 hours under stirring, followed by low-temperature centrifugation (4,000 rpm, 4°C for 30 minutes) to obtain an acid hydrolysate supernatant. A 40 wt% aqueous sodium hydroxide solution was added to the acid hydrolysate supernatant to adjust a pH value to 7.0 ± 0.5. Under stirring, 1-fold volume of 95 vol% ethanol (relative to a supernatant volume) was added to a resulting reaction system. An obtained mixture was maintained at -10°C for 24 hours and then centrifuged at a low temperature (4,000 rpm, 4°C for 30 minutes), and a formed precipitate was collected. The formed precipitate was dissolved in 500 mL of distilled water. Then, 1 L of 95 vol% ethanol was added thereto to obtain a mixture. The mixture was maintained at -10°C for 24 hours and then centrifuged at a low temperature (4,000 rpm, 4°C for 30 minutes), and a precipitate was collected. The precipitate was washed with 500 mL of 95 vol% ethanol, centrifuged at a low temperature (4,000 rpm, 4°C for 30 minutes), and a solid was collected. The solid was dissolved in 500 mL of distilled water and freeze-dried to obtain 4 g of a crude HPS.

The 4 g of crude HPS was loaded onto a DEAE cellulose column (10 cm × 4 cm) pre-equilibrated with a 0.1 mol/L HAc-NaAc buffer (pH=6). The DEAE cellulose column was successively washed with 5 L of a HAc-NaAc buffer (pH=6) containing 0.5 mol/L NaCl and 2 L of a HAc-NaAc buffer (pH=6) containing 1 mol/L NaCl. The DEAE cellulose column had a flow rate of 50 mL/minute, and 500 mL was collected per fraction. The fractions each were analyzed using HPLC methods for a molecular weight and a molecular weight distribution. Fractions containing chromatographic peaks corresponding to a weight-average molecular weight between 90,000 and 130,000 were pooled. 1-fold volume of 95 vol% ethanol (relative to a pooled effluent volume) was added to pooled fractions, maintained at -10°C for 24 hours and then centrifuged at a low temperature (4,000 rpm, 4°C for 30 minutes), and a resulting precipitate was collected. The resulting precipitate was dissolved in distilled water and concentrated to one-half an original volume using an ultrafiltration membrane with a molecular weight cut-off of 10,000. Distilled water was added to restore the original volume, and a resulting solution was ultrafiltered again to one-half the original volume for the first time. Distilled water was added to restore the original volume, and an obtained solution was ultrafiltered again to one-half the original volume for the second time. Distilled water was added to restore the original volume to obtain a solution, and the solution was ultrafiltered again to one-half the original volume for the third time. A finally obtained concentrate was collected and freeze-dried to obtain 2.5 g of a purified HPS (with sodium as a cation). Following the above procedure, multiple batches of parallel samples were continuously prepared. A sample from batch 202401 was used in subsequent examples. Tests showed that the sample from the batch 202401 had a weight-average molecular weight of 108,791 Daltons, a PDI of 1.32, a molar ratio of glucuronic acid, N-acetyl-D-galactosamine, and fucose being 1 : 1.04 : 0.67, sodium ions having a mass percentage of 9.24%, and sulfate groups having a mass percentage of 27.3% as determined by ion chromatography.

### Example 2 Establishment of a method for potency determination of an HPS reference standard

The pharmacological action of an HPS primarily manifests as that of a non-heparin-like anticoagulant drug. A mechanism of action involves selective inhibition of a terminal rate-limiting enzyme of an endogenous coagulation pathway, an "Intrinsic Factor Xase (Intrinsic Tenase, FIXa-FVIIIa-PL-Ca²⁺ complex, iFXase)".

Since a standard for an FVIII is readily available, an FVIII activity assay kit (FVIII:C) was used to conduct potency determination by assaying inhibitory activity of the HPS against the iFXase. Under assay conditions, an amount of the HPS that inhibited the iFXase activity required for formation of 1 IU of the FVIII was defined as one potency unit of the HPS.

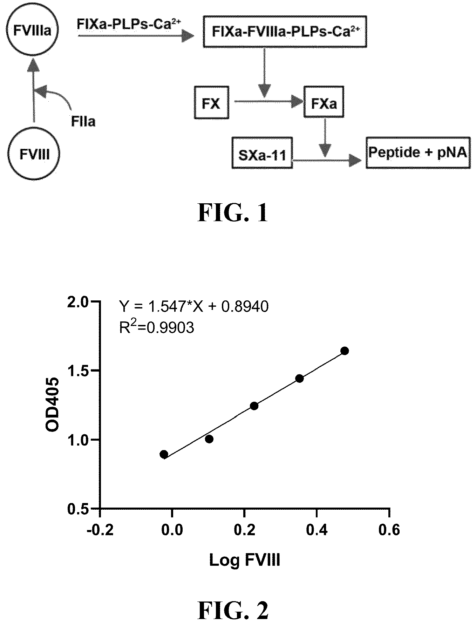

The detection mechanism of the FVIII activity assay kit was as follows: in a presence of phospholipid (PLP) and calcium ions, the FVIII was activated by thrombin to form an FVIIIa. The FVIIIa, an FIXa, the phospholipid, and the calcium ions formed an enzyme complex (iFXase), which subsequently activated an FX. A generated FXa hydrolyzed a chromogenic substrate SXa-11, releasing p-nitroaniline (pNA). The pNA exhibited light absorption at 405 nm, and an amount of the pNA was directly proportional to an absorbance value at 405 nm (OD₄₀₅). The activity of the enzyme complex is reflected by measuring a pNA content at 405 nm using a microplate reader. A logarithm of an FVIII concentration was proportional to the OD₄₀₅. A mass of the HPS that inhibited the enzyme complex activity required for the formation of 1 IU of the FVIII was defined as one potency unit of the HPS. FIG. 1 shows a schematic diagram of the detection mechanism of the FVIII activity assay kit.

A specific method for determination was performed as follows:

### 1. Instruments and Materials

The instruments and reagents required for an experiment are listed in Tables 1 and 2.

**Table 1 Instruments required for experiment**

| Name | Model/specification | Manufacturer |
|---|---|---|
| Microplate reader | BioTeK Synergy H1 | Agilent |
| Pipette | 1000 µL | Eppendorf |
| Pipette | 200 µL | Eppendorf |
| Pipette | 20 µL | Eppendorf |
| Pipette | 10-100 µL Eight-channel pipette | Eppendorf |
| Electronic multi-channel dispensing pipette | 1 µL-50 mL Multipette^{®} E3x | Eppendorf |
| Electronic balance | ME104T/02 | Mettler |
| Constant-temperature water bath | WB20 | Salvis LAB |

**Table 2 Reagents and materials required for experiment**

| Name | Batch No. | Trait | Specification | Source |
|---|---|---|---|---|
| HPS | 202401 | Freeze-dried powder | / | Harbin Hongdoushan Biopharmaceutical Co., Ltd., China |
| Human coagulation factor FVIII | 280016-202103 | Freeze-dried powder | 4.9 IU | National Institutes for Food and Drug Control, China |
| FVIII Activity Assay Kit | 221402 | - | BIOPHEN FVllI:C | BestopBio, Beijing, China |

### 2. Experimental Methods

### 2.1 FVIII Complex Activity Standard Curve

### 1) Preparation of kit solutions:

Ultrapure water (2.5 mL per vial) was added to R1 (FX), R2 (FIXa, FIIa, phospholipid, Tris-HCl, CaCl₂), and R3 (SXa-11) separately, to obtain an R1 (FX) solution, an R2 (activation reagent) solution, and an R3 (chromogenic substrate) solution. A concentration of the R1 solution was 50 nM. A R2 solution contained the FIXa at a concentration of 60 nM, the FIIa at a concentration of 47 nM, the phospholipid at a concentration of 0.32 mg/mL, and the CaCl₂ at a concentration of 12 mM. A concentration of the R3 solution was 8.4 mM. An R4 (Tris-BSA Buffer) was used for diluting an FVIII standard solution.

### 2) Preparation of FVIII Standard Solution:

An FVIII (4.9 IU/vial) was taken, and 1.225 mL of an R4 reagent was quantitatively aspirated to dissolve the FVIII, preparing a 4 IU/mL FVIII stock solution. From the 4 IU/mL FVIII stock solution, 1000 µL was taken, and 333 µL of the R4 was added thereto, to prepare a 3 IU/mL FVIII standard solution. From the 3 IU/mL FVIII standard solution, 600 µL was taken, and 200 µL of the R4 was added thereto, to prepare a 2.25 IU/mL FVIII standard solution. From the 2.25 IU/mL FVIII standard solution, 600 µL was taken, and 200 µL of the R4 was added thereto, to prepare a 1.6875 IU/mL FVIII standard solution. From the 1.6875 IU/mL FVIII standard solution, 600 µL was taken, and 200 µL of the R4 was added thereto, to prepare a 1.265625 IU/mL FVIII standard solution. From the 1.265625 IU/mL FVIII standard solution, 600 µL was taken, and 200 µL of the R4 was added thereto, to prepare a 0.94922 IU/mL FVIII standard solution.

### 3) Preparation of stop solution:

Citric acid (1 g) was weighed and dissolved in 50 mL of ddH₂O to prepare a 0.02 g/mL citric acid stop solution.

### 4) Determination of FVIII Complex Activity Standard Curve:

(1) 20 µL of ddH₂O was added to each of 5 test wells in a 96-well plate.
(2) 20 µL of different concentrations of the FVIII standard solution were added sequentially to the aforementioned test wells, followed by 20 µL of the R2 solution to each well. After addition, the 96-well plate was incubated at 37°C for 15 minutes.
(3) 20 µL of the R1 solution was added to each well, followed by incubation at 37°C for 1 minute.
(4) 20 µL of the R3 solution was added to each well, followed by incubation at 37°C for 15 minutes. Then, 30 µL of the stop solution was added, and an absorbance at 405 nm (OD₄₀₅) was measured using a microplate reader.
(5) The FVIII complex activity standard curve was plotted with a logarithm of an FVIII concentration as an abscissa and the OD₄₀₅ as an ordinate.

### 2.2 Polysaccharide Activity Potency Determination

### 1) Preparation of kit solutions:

Ultrapure water (2.5 mL per vial) was added to R1 (FX), R2 (FIXa, FIIa, phospholipid, Tris-HCl, CaCl₂), and R3 (SXa-11) separately, to obtain an R1 (FX) solution, an R2 (activation reagent) solution, and an R3 (chromogenic substrate) solution. An R4 (Tris-BSA Buffer) was used for diluting an FVIII standard solution. A preparation method and concentrations were the same as those for the kit solutions in section 2.1 for the FVIII complex activity standard curve.

### 2) Preparation of 3 IU FVIII Solution:

An FVIII (4.9 IU/vial) was taken, and 1.225 mL of an R4 reagent was quantitatively aspirated to dissolve the FVIII, preparing a 4 IU/mL FVIII solution. From the 4 IU/mL FVIII solution, 1000 µL was taken, and 333 µL of the R4 was added thereto, to prepare a 3 IU/mL FVIII solution.

### 3) Preparation of Polysaccharide Stock Solutions and Polysaccharide Working Solutions:

25 mg of a polysaccharide reference substance was exactly weighed and dissolved in 10 mL of ddH₂O to prepare a polysaccharide stock solution 1 (2.5 mg/mL). Then, 100 µL of the polysaccharide stock solution 1 was aspirated, and 9900 µL of ddH₂O was added thereto, to prepare a polysaccharide stock solution 2 (25 µg/mL). Next, 100 µL of the polysaccharide stock solution 2 was aspirated, and 4900 µL of ddH₂O was added thereto, to prepare a polysaccharide working solution 1 (500 ng/mL). Subsequently, 900 µL of the polysaccharide working solution 1 was aspirated, and 100 µL of ddH₂O was added thereto, to prepare a polysaccharide working solution 2 (450 ng/mL). Then, 900 µL of the polysaccharide working solution 2 was aspirated, and 100 µL of ddH₂O was added thereto, to prepare a polysaccharide working solution 3 (405 ng/mL). Next, 900 µL of the polysaccharide working solution 3 was aspirated, and 100 µL of ddH₂O was added thereto, to prepare a polysaccharide working solution 4 (364.5 ng/mL). Finally, 900 µL of the polysaccharide working solution 4 was aspirated, and 100 µL of ddH₂O was added thereto, to prepare a polysaccharide working solution 5 (328.05 ng/mL).

### 4) Preparation of Stop Solution:

Citric acid (1 g) was weighed and dissolved in 50 mL of ddH₂O to prepare a 0.02 g/mL citric acid stop solution.

### 5) Determination of Polysaccharide Activity:

(1) 20 µL of ddH₂O was added to a blank test well in a 96-well plate, and 20 µL of different concentrations of the polysaccharide working solutions were added sequentially to polysaccharide test wells.
(2) 20 µL of the 3 IU/mL FVIII solution was added sequentially to the aforementioned test wells, followed by 20 µL of the R2 to each well. After addition, the 96-well plate was incubated at 37°C for 15 minutes.
(3) 20 µL of the R1 solution was added to each well, followed by incubation at 37°C for 1 minute.
(4) 20 µL of the R3 solution was added to each well, followed by incubation at 37°C for 15 minutes. Then, 30 µL of the stop solution was added, and an absorbance at 405 nm (OD₄₀₅) was measured using a microplate reader.
(5) Absorbance values were substituted into the FVIII complex activity standard curve to calculate a residual FVIII concentration. For convenience of calculation, a regression equation was plotted with a reciprocal of a polysaccharide sample amount multiplied by 50 as an abscissa and a logarithm of a residual FVIII units multiplied by 50 as an ordinate.
(6) An initial FVIII concentration was 3 IU/mL, and an addition volume of both the FVIII and polysaccharide solutions was 20 µL. For convenience of calculation, data were scaled up by a factor of 50, calculating as if an FVIII addition volume was 1 mL. That is, an amount of the FVIII added to a reaction system was 3 IU. An amount of an HPS required to inhibit 1 IU, resulting in a residual of 2 IU, was defined as one potency unit of the HPS.

### 3. Experimental Results:

The following experimental data were obtained from two independent tests conducted separately by two experimental analysts. Results are as follows:

### 3.1 Experimenter A - First Experimental Results

### 1) FVIII Complex Activity Standard Curve

Test results for FVIII complex activity at different unit concentrations are shown in Table 3.

**Table 3 FVIII Complex Enzyme Activity Results**

| | | | | | |
|---|---|---|---|---|---|
| FVIII (IU/mL) | 0.94921875 | 1.265625 | 1.6875 | 2.25 | 3 |
| OD₄₀₅ | 0.895 | 1.005 | 1.243 | 1.442 | 1.643 |
| Log (FVIII) | -0.022633692 | 0.102305045 | 0.227243782 | 0.352182518 | 0.477121255 |

Based on measured experimental data, a standard curve was plotted with a logarithm of an FVIII concentration as an abscissa and an absorbance OD₄₀₅ as an ordinate. c result is shown in FIG. 2.

### 2) Determination Results of Polysaccharide Activity

The determination results for the polysaccharide activity at different concentrations are shown in Table 4. Based on measured experimental data, absorbance values of polysaccharide working solutions were substituted into an FVIII complex enzyme activity standard curve to calculate a logarithm of a residual FVIII concentration. Results are shown in Table 4.

**Table 4 Polysaccharide Test Activity Results**

| | | | | | |
|---|---|---|---|---|---|
| Polysaccharide working solution (µg/mL) | 0.32805 | 0.3645 | 0.405 | 0.45 | 0.5 |
| OD₄₀₅ | 0.421 | 0.328 | 0.278 | 0.227 | 0.173 |
| Log(FVIII) | -0.305753 0 | -0.365869 4 | -0.398190 0 | -0.431157 0 | -0.4660633 |

An initial FVIII concentration was 3 IU/mL, and an addition volume for both an FVIII and polysaccharide solutions was 20 µL. Data were scaled up by a factor of 50, calculating as if an amount of the FVIII added to a reaction system was 3 IU. Results are shown in Table 5 (Experimenter A - First Experimental Results).

**Table 5 Potency Determination Data**

| | Sample concentra tion (µg/mL) | Samp le amou nt (µg) | OD₄₀₅ | Logarithm of residual concentrati on (IU/mL) | Residual concentratio n (IU/mL) | Residual activity unit IU | Sample amount µg×50 | Residual activity unit IU×50 | 1/Sample amount (×50) | log residual activity unit (×50) |
|---|---|---|---|---|---|---|---|---|---|---|
| Expe rimen ter A First Expe rimen tal Resul ts | 0.32805 | 0.006 561 | 0.421 | -0.305753 | 0.494592 | 0.009892 | 0.32805 | 0.494592 | 3.048315 806 | -0.305753 |
| | 0.3645 | 0.007 29 | 0.328 | -0.3658694 | 0.430656 | 0.008613 | 0.3645 | 0.430656 | 2.743484 225 | -0.3658694 |
| | 0.405 | 0.008 1 | 0.278 | -0.39819 | 0.39977 | 0.007995 | 0.405 | 0.39977 | 2.469135 802 | -0.39819 |
| | 0.45 | 0.009 | 0.227 | -0.431157 | 0.370547 | 0.007411 | 0.45 | 0.370547 | 2.222222 222 | -0.431157 |
| | 0.5 | 0.01 | 0.173 | -0.4660633 | 0.34193 | 0.006839 | 0.5 | 0.34193 | 2 | -0.4660633 |

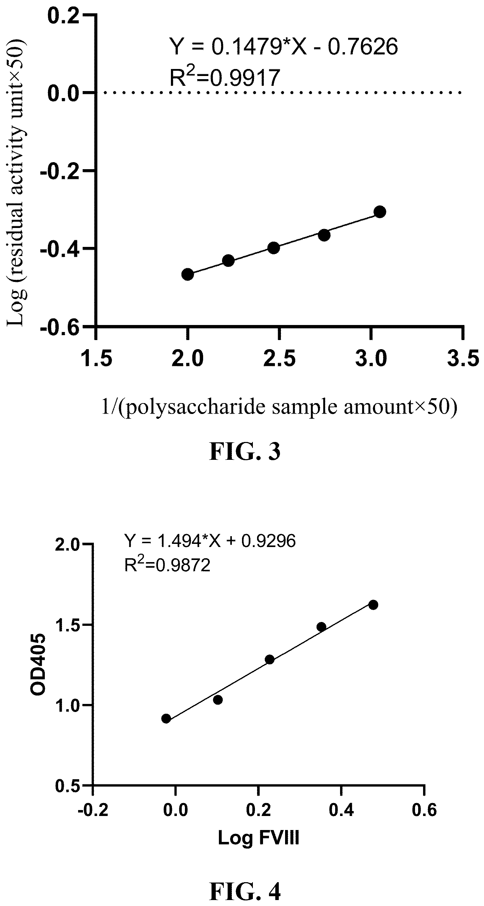

A weight of an HPS required to inhibit 1 IU, resulting in a residual of 2 IU (based on 3 IU of an FVIII added to a reaction system), was defined as one potency unit (i.e., 1 IU) of the HPS. Based on the results in Table 5, a regression equation was established with a logarithm of (residual activity units × 50) as an ordinate and the 1/(polysaccharide sample amount × 50) as an abscissa. A result is shown in FIG. 3.

According to the regression equation in FIG. 3, calculation for a residual activity unit of 2 IU (i.e., inhibiting 1 IU) was conducted. Log 2IU = 0.301029996 was substituted into the regression equation (i.e., 0.1479X - 0.7626 = 0.301029996), calculating a reciprocal of the sample amount (X) as 7.191548 µg⁻¹, meaning the sample amount was 0.139052115 µg. For convenience of expression, a potency of the HPS was expressed as a number of potency units per unit mass (mg) of polysaccharide. A calculated potency of an HPS reference standard was 7,192 IU/mg.

### 3.2 Experimenter A - Second Experimental Results

### 1) FVIII Complex Activity Standard Curve

Test results for FVIII complex activity at different unit concentrations are shown in Table 6.

**Table 6 FVIII Complex Enzyme Activity Results**

| | | | | | |
|---|---|---|---|---|---|
| FVIII (IU/mL) | 0.94921875 | 1.265625 | 1.6875 | 2.25 | 3 |
| OD₄₀₅ | 0.917 | 1.033 | 1.285 | 1.487 | 1.623 |
| Log (FVIII) | -0.022633692 | 0.102305045 | 0.227243782 | 0.352182518 | 0.477121255 |

Based on measured experimental data, a standard curve was plotted with a logarithm of an FVIII concentration as an abscissa and an absorbance OD₄₀₅ as an ordinate. A result is shown in FIG. 4.

### 2) Determination Results of Polysaccharide Activity

The determination results for the polysaccharide activity at different unit concentrations are shown in Table 7. Based on measured experimental data, absorbance values of polysaccharide working solutions were substituted into an FVIII complex enzyme activity standard curve to calculate a logarithm of a residual FVIII concentration. Results are shown in Table 7.

**Table 7 Polysaccharide Activity Test Results**

| | | | | | |
|---|---|---|---|---|---|
| Polysaccharide working solution (µg/mL) | 0.32805 | 0.3645 | 0.405 | 0.45 | 0.5 |
| OD₄₀₅ | 0.418 | 0.328 | 0.27 | 0.227 | 0.182 |
| Log FVIII concentration | -0.34244 | -0.40268 | -0.441499 | -0.47028 | -0.5004 |

An initial FVIII concentration was 3 IU/mL, and an addition volume for both FVIII and polysaccharide solutions was 20 µL. Data were scaled up by a factor of 50, calculating as if an amount of the FVIII added to a reaction system was 3 IU. Results are shown in Table 8 (Experimenter A - Second Experimental Results).

**Table 8 Potency Determination Data**

| | Sample concent ration (µg/mL ) | Sample amount (µg) | OD₄₀₅ | Logarithm of residual concentrati on (IU/mL) | Residual concentrati on (IU/mL) | Residual activity unit IU | Sample amount µg×50 | Residual activity unit IU×50 | 1/Sample amount (×50) | log residual activity unit (×50) |
|---|---|---|---|---|---|---|---|---|---|---|
| Expe rimen ter A Seco nd Expe rimen tal Resul ts | 0.32805 | 0.0065 61 | 0.418 | -0.34244 | 0.454532 | 0.009091 | 0.32805 | 0.454532 | 3.048315 806 | -0.34244 |
| | 0.3645 | 0.0072 9 | 0.328 | -0.40268 | 0.395661 | 0.007913 | 0.3645 | 0.395661 | 2.743484 225 | -0.40268 |
| | 0.405 | 0.0081 | 0.27 | -0.4415 | 0.361827 | 0.007237 | 0.405 | 0.361827 | 2.469135 802 | -0.4415 |
| | 0.45 | 0.009 | 0.227 | -0.47028 | 0.338625 | 0.006772 | 0.45 | 0.338625 | 2.222222 222 | -0.47028 |
| | 0.5 | 0.01 | 0.182 | -0.5004 | 0.315935 | 0.006319 | 0.5 | 0.315935 | 2 | -0.5004 |

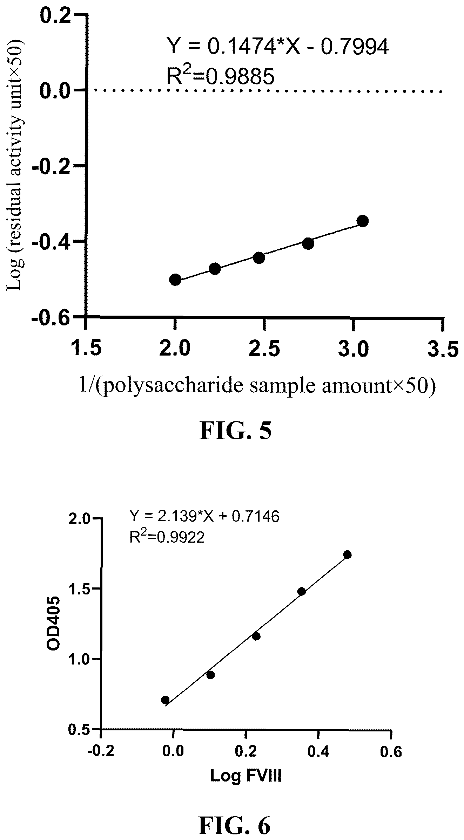

A weight of an HPS required to inhibit 1 IU, resulting in a residual of 2 IU (based on 3 IU of an FVIII added to a reaction system), was defined as one potency unit (i.e., 1 IU) of the HPS. Based on the results in Table 8, a regression equation was established with a logarithm of (residual activity units × 50) as an ordinate and the 1/(polysaccharide sample amount × 50) as an abscissa. A result is shown in FIG. 5.

For a residual activity unit of 2 IU, Log 2IU = 0.301029996 was substituted into the regression equation, calculating a reciprocal of the sample amount as 7.465604 µg⁻¹, meaning the sample amount was 0.133948 µg. For convenience of expression, a potency of the HPS was expressed as a number of potency units per unit mass (mg) of polysaccharide. A calculated potency of a polysaccharide reference substance was 7,466 IU/mg.

### 3.3 Experimenter B - First Experimental Results

### 1) FVIII Complex Activity Standard Curve

Test results for FVIII complex activity at different unit concentrations are shown in Table 9.

**Table 9 FVIII Complex Enzyme Activity Results**

| | | | | | |
|---|---|---|---|---|---|
| FVIII (IU/mL) | 0.94921875 | 1.265625 | 1.6875 | 2.25 | 3 |
| OD₄₀₅ | 0.711 | 0.891 | 1.165 | 1.487 | 1.749 |
| Log (FVIII) | -0.022633692 | 0.102305045 | 0.227243782 | 0.352182518 | 0.477121255 |

Based on measured experimental data, a standard curve was plotted with a logarithm of an FVIII concentration as an abscissa and an absorbance OD₄₀₅ as an ordinate. A result is shown in FIG. 6.

### 2) Determination Results of Polysaccharide Activity

The determination results for the polysaccharide activity at different unit concentrations are shown in Table 10. Based on measured experimental data, absorbance values of polysaccharide working solutions were substituted into an FVIII complex enzyme activity standard curve to calculate a logarithm of a residual FVIII concentration. Results are shown in Table 10.

**Table 10 Polysaccharide Activity Test Results**

| Polysaccharide working solution (µg/mL) | 0.32805 | 0.3645 | 0.405 | 0.45 | 0.5 |
|---|---|---|---|---|---|
| OD₄₀₅ | 0.373 | 0.326 | 0.238 | 0.211 | 0.166 |
| Log FVIII concentration | -0.1597 | -0.18167 | -0.2228144 | -0.23544 | -0.25647 |

An initial FVIII concentration was 3 IU/mL, and an addition volume for both an FVIII and polysaccharide solutions was 20 µL. Data were scaled up by a factor of 50, calculating as if an amount of the FVIII added to a reaction system was 3 IU. Results are shown in Table 11 (Experimenter B - First Experimental Results).

**Table 11 Potency Determination Data**

| | Sample concentr ation (µg/mL) | Sample amount (µg) | OD₄₀₅ | Logarithm of residual concentrat ion (IU/mL) | Residual concentrat ion (IU/mL) | Residual activity unit IU | Sample amount µg×50 | Residual activity unit IU×50 | 1/Sample amount (×50) | log residual activity unit (×50) |
|---|---|---|---|---|---|---|---|---|---|---|
| Expe rimen ter B First Expe rimen tal Resul ts | 0.32805 | 0.006561 | 0.373 | -0.1597 | 0.692309 | 0.013846 | 0.32805 | 0.692309 | 3.0483158 06 | -0.1597 |
| | 0.3645 | 0.00729 | 0.326 | -0.18167 | 0.658158 | 0.013163 | 0.3645 | 0.658158 | 2.7434842 25 | -0.18167 |
| | 0.405 | 0.0081 | 0.238 | -0.222814 | 0.598667 | 0.011973 | 0.405 | 0.598667 | 2.4691358 02 | -0.222814 |
| | 0.45 | 0.009 | 0.211 | -0.23544 | 0.581514 | 0.01163 | 0.45 | 0.581514 | 2.2222222 22 | -0.23544 |
| | 0.5 | 0.01 | 0.166 | -0.25647 | 0.554026 | 0.011081 | 0.5 | 0.554026 | 2 | -0.25647 |

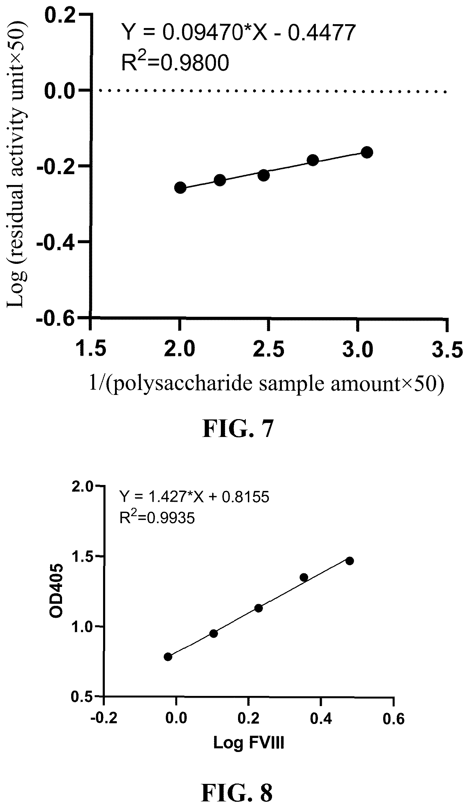

A weight of an HPS required to inhibit 1 IU, resulting in a residual of 2 IU (based on 3 IU of an FVIII added to a reaction system), was defined as one potency unit (i.e., 1 IU) of the HPS. Based on the results in Table 11, a regression equation was established with a logarithm of (residual activity units × 50) as an ordinate and the 1/(polysaccharide sample amount × 50) as an abscissa. A result is shown in FIG. 7.

For a residual activity unit of 2 IU, Log 2IU = 0.301029996 was substituted into the regression equation, calculating a reciprocal of the sample amount as 7.906336 µg⁻¹, meaning the sample amount was 0.126481 µg. For convenience of expression, a potency of the HPS was expressed as a number of potency units per unit mass (mg) of polysaccharide. A calculated potency of a polysaccharide reference substance was 7,906 IU/mg.

### 3.4 Experimenter B - Second Experimental Results

### 1) FVIII Complex Activity Standard Curve

Test results for FVIII complex activity at different unit concentrations are shown in Table 12.

**Table 12 FVIII Complex Enzyme Activity Results**

| | | | | | |
|---|---|---|---|---|---|
| FVIII (IU/mL) | 0.94921875 | 1.265625 | 1.6875 | 2.25 | 3 |
| OD₄₀₅ | 0.783 | 0.953 | 1.134 | 1.356 | 1.473 |
| Log (FVIII) | -0.022633692 | 0.102305045 | 0.227243782 | 0.352182518 | 0.477121255 |

Based on measured experimental data, a standard curve was plotted with a logarithm of an FVIII concentration as an abscissa and an absorbance OD₄₀₅ as an ordinate. A result is shown in FIG. 8.

### 2) Determination Results of Polysaccharide Activity

The determination results for the polysaccharide activity at different unit concentrations are shown in Table 13. Based on measured experimental data, absorbance values of polysaccharide working solutions were substituted into an FVIII complex enzyme activity standard curve to calculate a logarithm of a residual FVIII concentration. Results are shown in Table 13.

**Table 13 Polysaccharide Activity Test Results**

| | | | | | |
|---|---|---|---|---|---|
| Polysaccharide working solution (µg/mL) | 0.32805 | 0.3645 | 0.405 | 0.45 | 0.5 |
| OD₄₀₅ | 0.446 | 0.357 | 0.291 | 0.227 | 0.203 |
| Log FVIII concentration | -0.25893 | -0.3213 | -0.36755 | -0.4124 | -0.42922 |

An initial FVIII concentration was 3 IU/mL, and an addition volume for both an FVIII and polysaccharide solutions was 20 µL. Data were scaled up by a factor of 50, calculating as if an amount of the FVIII added to a reaction system was 3 IU. Results are shown in Table 14 (Experimenter B - Second Experimental Results).

**Table 14 Potency Determination Data**

| | Sample concentr ation (µg/mL) | Sample amount (µg) | OD₄₀₅ | Logarith m of residual concentr ation (IU/mL) | Residual concentrati on (IU/mL) | Residual activity unit IU | Sample amount µg×50 | Residual activity unit IU×50 | 1/Sample amount (×50) | log residual activity unit (×50) |
|---|---|---|---|---|---|---|---|---|---|---|
| Expe rimen ter B Seco nd Expe rimen tal Resul ts | 0.32805 | 0.00656 1 | 0.446 | -0.25893 | 0.55089 | 0.011018 | 0.32805 | 0.55089 | 3.048315 806 | -0.25893 |
| | 0.3645 | 0.00729 | 0.357 | -0.3213 | 0.477196 | 0.009544 | 0.3645 | 0.477196 | 2.743484 225 | -0.3213 |
| | 0.405 | 0.0081 | 0.291 | -0.36755 | 0.428989 | 0.00858 | 0.405 | 0.428989 | 2.469135 802 | -0.36755 |
| | 0.45 | 0.009 | 0.227 | -0.4124 | 0.386898 | 0.007738 | 0.45 | 0.386898 | 2.222222 222 | -0.4124 |
| | 0.5 | 0.01 | 0.203 | -0.42922 | 0.372201 | 0.007444 | 0.5 | 0.372201 | 2 | -0.42922 |

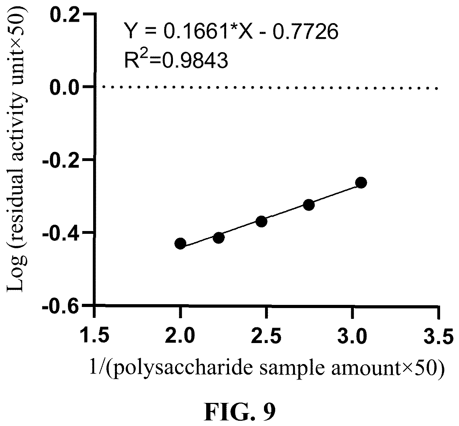

A weight of an HPS required to inhibit 1 IU, resulting in a residual of 2 IU (based on 3 IU of an FVIII added to a reaction system), was defined as one potency unit (i.e., 1 IU) of the HPS. Based on the results in Table 14, a regression equation was established with a logarithm of (residual activity units × 50) as an ordinate and the 1/(polysaccharide sample amount × 50) as an abscissa. A result is shown in FIG. 9.

For a residual activity unit of 2 IU, Log 2IU = 0.301029996 was substituted into the regression equation, calculating a reciprocal of the sample amount as 6.463757 µg⁻¹, meaning the sample amount was 0.154709 µg. For convenience of expression, a potency of the HPS was expressed as a number of potency units per unit mass (mg) of polysaccharide. A calculated potency of a polysaccharide reference substance was 6,464 IU/mg.

### 3.5 HPS Potency Determination Result

In summary, the potencies of the HPS from the four experimental results (two experimenters, two tests each) were 7,192 IU/mg, 7,466 IU/mg, 7,906 IU/mg, and 6,464 IU/mg, respectively. A mean of the four potencies was 7,257 IU/mg. Based on this, the potency of the HPS was determined to be 7,257 IU/mg.

The above descriptions are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. A method for potency determination of a haina polysaccharide (HPS), comprising the following steps:
obtaining an HPS activity curve by a p-nitroaniline chromogenic assay, wherein the HPS activity curve uses a reciprocal of an HPS weight as an abscissa and a logarithm of a residual Factor VIII (FVIII) activity unit as an ordinate; and
defining a weight of the HPS that inhibits an Intrinsic Factor Xase (iFXase) activity required for formation of 1 IU of an FVIII as one potency unit, and obtaining a potency of the HPS from the HPS activity curve; wherein
the HPS is a fucosylated chondroitin sulfate polysaccharide; the HPS has a weight-average molecular weight of 90,000 to 130,000, and has a monosaccharide composition comprising glucuronic acid, N-acetyl-D-galactosamine, and fucose, wherein a molar ratio of the glucuronic acid, the N-acetyl-D-galactosamine, and the fucose is in a range of 1 : 0.8-1.2 : 0.5-0.8; and a sulfate group in the HPS has a mass percentage of 25% to 40%.

2. The method for potency determination of the HPS of claim 1, wherein the obtaining the HPS activity curve comprises:
preparing HPS working solutions with gradient concentrations;
mixing the HPS working solutions, an FVIII solution, and an activation reagent solution, and conducting a first incubation to obtain a first incubation mixture, wherein the activation reagent solution comprises a Factor IXa (FIXa), a Factor IIa (Thrombin) (FIIa), a phospholipid, a buffer, and CaCl₂;
mixing the first incubation mixture with a Factor X (FX) solution, and conducting a second incubation to obtain a second incubation mixture;
mixing the second incubation mixture with a chromogenic substrate solution, conducting a third incubation, adding a stop solution, and measuring an absorbance of a resulting mixture at 405 nm to obtain an absorbance value, wherein a chromogenic substrate in the chromogenic substrate solution is SXa-11 (Sequence: Suc-Ile-Glu-(Pip)Gly-Arg-pNA, HCl); and
substituting the absorbance value into an FVIII complex activity standard curve, calculating the residual FVIII activity unit, and plotting the HPS activity curve using the reciprocal of the HPS weight as the abscissa and the logarithm of the residual FVIII activity unit as the ordinate; wherein the FVIII complex activity standard curve uses a logarithm of an FVIII concentration as an abscissa and the absorbance value as an ordinate.

3. The method for potency determination of the HPS of claim 2, wherein the first incubation is conducted at 37°C for 5 minutes to 20 minutes.

4. The method for potency determination of the HPS of claim 2, wherein the second incubation is conducted at 37°C for 1 minute to 10 minutes.

5. The method for potency determination of the HPS of claim 2, wherein the third incubation is conducted at 37°C for 10 minutes to 30 minutes.

6. The method for potency determination of the HPS of claim 2, wherein the stop solution is a citric acid solution having a concentration of 0.01 g/mL to 0.1 g/mL.

7. The method for potency determination of the HPS of claim 2, wherein the HPS working solutions each have a concentration of 328 ng/mL to 500 ng/mL.

8. The method for potency determination of the HPS of claim 2, wherein the buffer in the activation reagent solution is Tris(hydroxymethyl)aminomethane hydrochloride (Tris-HCl).

9. The method for potency determination of the HPS of claim 2, wherein the FX solution has a concentration of 40 nM to 60 nM.

10. The method for potency determination of the HPS of claim 2, wherein the activation reagent solution comprises the FIXa at a concentration of 40 nM to 80 nM, the FIIa at a concentration of 30 nM to 60 nM, the phospholipid at a concentration of 0.1 mg/mL to 0.6 mg/mL, and the CaCl₂ at a concentration of 5 mM to 15 mM.

11. The method for potency determination of the HPS of claim 2, wherein the chromogenic substrate solution has a concentration of 4 mM to 12 mM.

12. The method for potency determination of the HPS of claim 2, wherein a volume ratio of the HPS working solutions to the FVIII solution is in a range of 1 : 1; a volume ratio of the HPS working solutions to the activation reagent solution is in a range of 1 : 1; a volume ratio of the HPS working solutions to the FX solution is in a range of 1 : 1; a volume ratio of the HPS working solutions to the chromogenic substrate solution is in a range of 1 : 1; and a volume ratio of the HPS working solutions to the stop solution is in a range of 2 : 3.

13. The method for potency determination of the HPS of claim 2, wherein the FVIII complex activity standard curve is obtained by a process comprising:
preparing FVIII standard solutions with gradient concentrations;
mixing the FVIII standard solutions, water, and the activation reagent solution, and conducting a fourth incubation to obtain a fourth incubation mixture, wherein the activation reagent solution comprises the FIXa, the FIIa, the phospholipid, the buffer, and the CaCl₂;
mixing the fourth incubation mixture with the FX solution, and conducting a fifth incubation to obtain a fifth incubation mixture;
mixing the fifth incubation mixture with the chromogenic substrate solution, conducting a sixth incubation, adding the stop solution, and measuring an absorbance of an obtained mixture at 405 nm to obtain the absorbance value, wherein the chromogenic substrate is the SXa-11; and
plotting the FVIII complex activity standard curve using the logarithm of the FVIII concentration as the abscissa and the absorbance value as the ordinate.

14. The method for potency determination of the HPS of claim 13, wherein the FVIII standard solutions each have a concentration of 0.95 IU/mL to 3 IU/mL.
